# EUROPEAN PATENT APPLICATION

(11) **EP 2 740 399 A1**
(43) Date of publication of application: **11.06.2014**
(21) Application number: 12872048.9
(22) Date of filing: 19.12.2012
(51) Int. Cl.: A61B 1/00

(54) **MEASUREMENT PROBE, BIO-OPTICAL MEASUREMENT APPARATUS AND BIO-OPTICAL MEASUREMENT SYSTEM**

(30) Priority: 22.03.2012 US 201261614202 P
(71) Applicant: Olympus Medical Systems Corp., Tokyo 151-0072 (JP); Olympus Corporation, Shibuya-ku Tokyo 151-0072 (JP)
(72) Inventor: KAMIMURA, Kenji, Tokyo 151-0072 (JP); GONO, Kazuhiro, Tokyo 151-0072 (JP); KOBAYASHI, Yoshimine, Tokyo 151-0072 (JP); SUGA, Takeshi, Tokyo 151-0072 (JP); KATAKURA, Masahiro, Tokyo 151-0072 (JP); ITO, Ryosuke, Tokyo 151-0072 (JP)
(74) Representative: Schicker, Silvia
(86) International application number: PCT/JP2012/082942
(87) International publication number: WO 2013/140689

(57) **Abstract**

Provided are a measurement probe, a bio-optical measurement apparatus, and a bio-optical measurement system, which are able to prevent, with a simple structure, erroneous connection. A measurement probe 3 has a fiber bundle formed by irregularly bundling a plurality of optical fibers and is detachably connected to a bio-optical measurement apparatus that performs optical measurement on a biological tissue. The measurement probe includes a recording medium 35 that records positional information and items to be examined of the biological tissue in association with each other. The positional information is related to each of positions of an illumination fiber that emits light from a light source 211 of the bio-optical measurement apparatus 2 as illumination light and of a first light-receiving fiber that receives returned light of the illumination light reflected and/or scattered at the biological tissue. The positions are in the fiber bundle at both end faces of each of the illumination fiber and the first light-receiving fiber.

## Description

### Field

The present invention relates to a measurement probe used in optical measurement of a biological tissue, a bio-optical measurement apparatus to which the measurement probe is connected, and a bio-optical measurement system. Background

Bio-optical measurement apparatuses are known, which irradiate illumination light to biological tissues and estimate characteristics of the biological tissues based on measurement values of detected light reflected or scattered from the biological tissues. The bio-optical measurement apparatuses are used in combination with endoscopes for observing organs such as digestive organs. As such a bio-optical measurement apparatus, a bio-optical measurement apparatus has been proposed, which employs LEBS (low-coherence enhanced backscattering) for detecting characteristics of a biological tissue by irradiating low coherence white light having a short spatial coherence length to the biological tissue from a distal end of an illumination fiber of a measurement probe and measuring an intensity distribution of scattered light beams of plural angles using plural light-receiving fibers (see Patent Literature 1).

Further, a bio-optical measurement apparatus has been proposed, which detects characteristics of a biological tissue using a measurement probe having a fiber bundle of which plural optical fibers are bundled together (see Patent Literature 2).

### Citation List

### Patent Literature

Patent Literature 1: National Publication of Japanese Translation of PCT Application No. 2009-537014
Patent Literature 2: National Publication of Japanese Translation of PCT Application No. 2003-511693

### Summary

### Technical Problem

However, since optical fibers have small diameters, much labor may be required to manufacture the optical fibers in such a way that positional relations between the fibers are equal to one another. Accordingly, measurement probes that are able to prevent, with a simple structure, erroneous connection have been desired.

The present invention has been made in view of the foregoing and an object of the invention is to provide a measurement probe, a bio-optical measurement apparatus, and a bio-optical measurement system, which are able to prevent, with a simple structure, erroneous connection. Solution to Problem

In order to solve the above-described problem and to achieve the object, a measurement probe according to the invention has a fiber bundle formed by irregularly bundling a plurality of optical fibers and is detachably connected to a bio-optical measurement apparatus that performs optical measurement to a biological tissue that is an object to be measured. The measurement probe includes a recording unit that records positional information and items to be examined of the biological tissue in association with each other. The positional information is related to each of positions of an illumination fiber that emits light from a light source of the bio-optical measurement apparatus as illumination light and of a light-receiving fiber that receives returned light of the illumination light reflected and/or scattered at the biological tissue. The positions are in the fiber bundle at both end faces of each of the illumination fiber and the light-receiving fiber.

According to the measurement probe of the invention, in the above invention, the recording unit is a recording medium that is attached to the fiber bundle and from which information is readable from outside.

According to the measurement probe of the invention, in the above invention, the fiber bundle is a light guide.

A bio-optical measurement apparatus according to the invention is a bio-optical measurement apparatus to which a measurement probe having a fiber bundle formed by irregularly bundling a plurality of optical fibers is connectable and which irradiates illumination light to a biological tissue through the measurement probe, receives returned light of the illumination light reflected and/or scattered at the biological tissue, and performs optical measurement of the biological tissue. The bio-optical measurement apparatus includes: a light source that generates the illumination light to be emitted to the biological tissue through the measurement probe; a light source driving unit that moves the light source in a predetermined direction; a sensor unit that receives the returned light through the measurement probe; a sensor driving unit that moves the sensor unit in a predetermined direction; and a control unit that moves, based on the measurement probe connected to the bio-optical measurement apparatus, the light source to a position of an illumination fiber that emits the illumination light to the biological tissue, and the sensor unit to a position of a light-receiving fiber that receives the returned light, by driving the light source driving unit and the sensor driving unit respectively, the position of the illumination fiber being in the fiber bundle at both end faces of the illumination fiber, the position of the light-receiving fiber being in the fiber bundle at both end faces of the light-receiving fiber.

According to the bio-optical measurement apparatus of the invention, in the above invention, the measurement probe includes a recording unit that records positional information and items to be examined of the biological tissue in association with each other. The positional information is related to each of positions of the illumination fiber and light-receiving fiber. The positions are in the fiber bundle at both end faces of each of the illumination fiber and the light-receiving fiber. The bio-optical measurement apparatus further includes a reading unit that reads the positional information from the recording unit, and the control unit drives each of the light source driving unit and the sensor driving unit based on the positional information read by the reading unit.

A bio-optical measurement system according to the invention includes: a measurement probe having a fiber bundle formed by irregularly bundling a plurality of optical fibers; and a bio-optical measurement apparatus to which the measurement probe is connectable and that irradiates illumination light to a biological tissue, receives returned light of the illumination light reflected and/or scattered at the biological tissue, and performs optical measurement of the biological tissue. The bio-optical measurement system includes: a light source that generates the illumination light to be emitted to the biological tissue through the measurement probe; a light source driving unit that moves the light source in a predetermined direction; a sensor unit that receives the returned light through the measurement probe; a sensor driving unit that moves the sensor unit in a predetermined direction; a recording unit that records positional information and items to be examined of the biological tissue in association with each other, the positional information being related to each of positions of an illumination fiber that emits light from the light source as illumination light and of a light-receiving fiber that receives the returned light, the positions being in the fiber bundle at both end faces of each of the illumination fiber and the light-receiving fiber; a reading unit that reads the positional information from the recording unit; and a control unit that moves, based on the positional information read by the reading unit, the light source to a position of an illumination fiber that emits the illumination light to the biological tissue, and the sensor unit to a position of a light-receiving fiber that receives the returned light, by driving the light source driving unit and the sensor driving unit respectively, the position of the illumination fiber being in the fiber bundle at both end faces of the illumination fiber, the position of the light-receiving fiber being in the fiber bundle at both end faces of the light-receiving fiber.

### Advantageous Effects of Invention

The present invention includes a recording unit that records positional information and items to be examined of the biological tissue in association with each other. The positional information is related to each of positions of an illumination fiber that emits light from a light source of a bio-optical measurement apparatus and of a light-receiving fiber that receives returned light reflected and/or scattered at a biological tissue. The positions are in a fiber bundle at both end faces of each of the illumination fiber and the light-receiving fiber. Therefore, it is possible to prevent, with a simple structure, erroneous connection.

### Brief Description of Drawings

FIG. 1 is a block diagram schematically illustrating a configuration of a bio-optical measurement system according to a first embodiment of the present invention.
FIG. 2 is a sectional view taken along a line A-A in FIG. 1.
FIG. 3 is a sectional view taken along a line B-B in FIG. 1.
FIG. 4 is a view schematically illustrating a recording medium attached to a measurement probe of the bio-optical measurement system according to the first embodiment of the present invention.
FIG. 5 is a flowchart illustrating an outline of a positioning process executed by the bio-optical measurement system according to the first embodiment of the present invention.
FIG. 6 is a block diagram schematically illustrating a configuration of a bio-optical measurement system according to a second embodiment of the present invention.
FIG. 7 is a view illustrating a configuration of a mask plate of a fiber selecting unit in the bio-optical measurement system according to the second embodiment of the present invention.
FIG. 8 is a view illustrating another configuration of the mask plate of the fiber selecting unit in the bio-optical measurement system according to the second embodiment of the present invention.
FIG. 9 is a view illustrating another configuration of the mask plate of the fiber selecting unit in the bio-optical measurement system according to the second embodiment of the present invention.
FIG. 10 is a flowchart illustrating an outline of a positioning process executed by the bio-optical measurement system according to the second embodiment of the present invention.

### Description of Embodiments

Preferable embodiments of a measurement probe, a bio-optical measurement apparatus, and a bio-optical measurement system according to the present invention will be described in detail with reference to the drawings. The present invention is not limited by these embodiments. In the description of drawings, like reference numerals denote like elements. Further, it is to be noted that the drawings are schematic, and relations between thicknesses and widths of each element, and ratios among elements are different from those of the actual. Among the drawings also, a same portion having relations or ratios of dimensions different from one another is included. A three-dimensional orthogonal coordinate system including a Z axis of which a positive direction is an upward direction in a vertical direction will be assumed in the description.

### (First Embodiment)

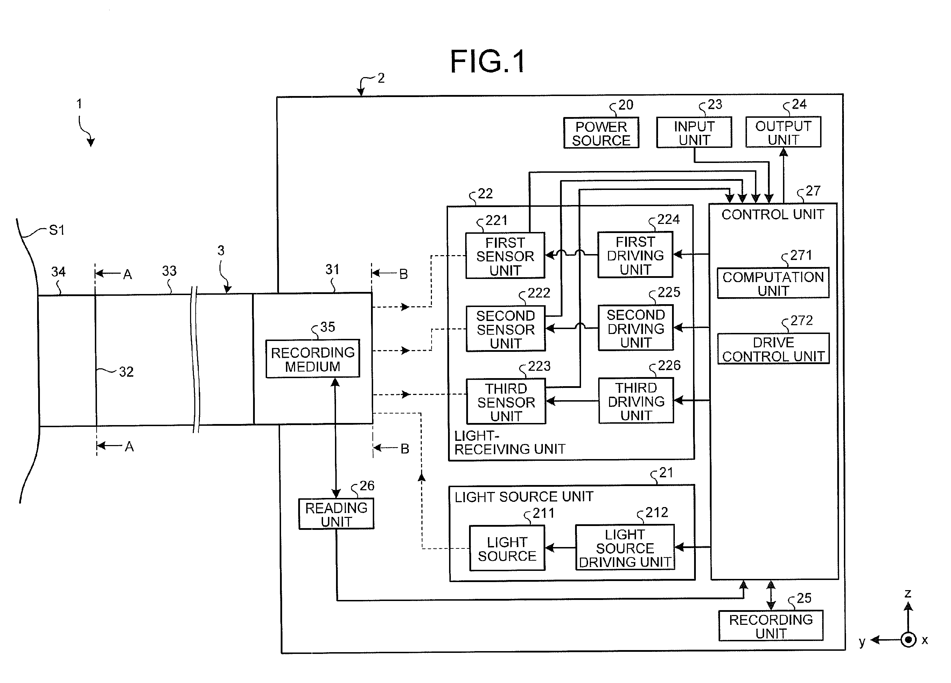

FIG. 1 is a block diagram schematically illustrating a configuration of a bio-optical measurement system according to a first embodiment of the present invention. The bio-optical measurement system illustrated in FIG. 1 includes a bio-optical measurement apparatus 2 that performs optical measurement with respect to an object S1 to be measured, which is a scatterer, such as a biological tissue to detect characteristics (properties) of the object S1 to be measured, and a measurement 3 probe for measurement, which is inserted into a subject. The object S1 to be measured is, for example, a biological tissue, a blood flow, an organ such as a stomach or a pancreas, or a mucous membrane.

The bio-optical measurement apparatus 2 will be described. The bio-optical measurement apparatus 2 includes a power source 20, a light source unit 21, a light-receiving unit 22, an input unit 23, an output unit 24, a recording unit 25, a reading unit 26, and a control unit 27. The power source 20 supplies electric power to each component of the bio-optical measurement apparatus 2.

The light source unit 21 supplies illumination light to a measurement probe 3 connected to the bio-optical measurement apparatus 2. The light source unit 21 includes a light source 211 and a light source driving unit 212.

The light source 211 is realized using an incoherent light source such as a white LED (light emitting diode), a xenon lamp, a tungsten lamp, or a halogen lamp, and as needed, one lens or plural lenses, e.g., a condensing lens or a collimator lens. The light source 211 supplies incoherent light, which is to be irradiated onto the object S1 to be measured and has at least one spectrum component, to an illumination fiber of the measurement probe 3 described later. The light source 211 is provided to be movable in a horizontal direction and/or a vertical direction in the bio-optical measurement apparatus 2.

The light source driving unit 212 is composed using a stepping motor, a DC motor, or the like, and by moving the light source 211 in a predetermined direction, e.g., in the horizontal direction (x axis) and/or in the vertical direction (z axis) under the control of the control unit 27, causes a position of illumination light (light beam) of the light source 211 to coincide with a position of the illumination fiber of the measurement probe 3 described later.

The light-receiving unit 22 receives and measures returned light of the illumination light, which is emitted from the measurement probe 3 and reflected and/or scattered by the object S1 to be measured. The light-receiving unit 22 includes a first sensor unit 221, a second sensor unit 222, a third sensor unit 223, a first driving unit 224, a second driving unit 225, and a third driving unit 226.

The first sensor unit 221 measures a spectrum component and an intensity distribution of the returned light of the illumination light, the returned light being emitted from a light-receiving fiber of the measurement probe 3 described later, and performs measurement of each wavelength. The first sensor unit 221 outputs results of the measurement to the control unit 27. The first sensor unit 221 is provided to be movable in the horizontal direction and/or vertical direction in the bio-optical measurement apparatus 2. The second sensor unit 222 and the third sensor unit 223 have the same configuration as the first sensor unit 221, so their descriptions will be omitted.

The first driving unit 224 is composed using a stepping motor, a DC motor, or the like, and by moving the first sensor unit 221 in a predetermined direction, e.g., in the horizontal direction and/or the vertical direction, under the control of the control unit 27, causes the position of the first sensor unit 221 to coincide with the position of the light-receiving fiber of the measurement probe 3 described later.

The second driving unit 225 has the same configuration as the first driving unit 224, and by moving the second sensor unit 222 in the horizontal direction and/or the vertical direction under the control of the control unit 27, causes the position of the second sensor unit 222 to coincide with the position of a light-receiving fiber of the measurement probe 3 described later.

The third driving unit 226 has the same configuration as the first driving unit 224, and by moving the third sensor unit 223 in the horizontal direction and/or the vertical direction under the control of the control unit 27, causes the position of the third sensor unit 223 to coincide with the position of a light-receiving fiber of the measurement probe 3 described later.

The input unit 23 is realized using a push-type switch, a keyboard, a touch panel, or the like and receives an input of an activating signal for instructing activation of the bio-optical measurement system 1 or an instruction signal for instructing an operation of other various types of operations, and outputs the received input to the control unit 27.

The output unit 24 is realized using a display of liquid crystal or organic EL (electroluminescence), a speaker, and the like and outputs information related to various processes in the bio-optical measurement system 1.

The recording unit 25 is realized using a volatile memory or a non-volatile memory and records therein various programs for operating the bio-optical measurement apparatus 2, and various data and various parameters used in an optical measurement process. The recording unit 25 temporarily records therein information that is being processed in the bio-optical measurement apparatus 2. The recording unit 25 records therein results of the measurement by the bio-optical measurement apparatus 2. The recording unit 25 may be composed using a memory card or the like installed from the outside of the bio-optical measurement apparatus 2.

The reading unit 26 reads information from a recording medium attached to a proximal end of the measurement probe 3 described later. The reading unit 26 is composed using, for example, a barcode reader, an RFID reader, or an IC chip reader, and outputs information, which has been read, to the control unit 27.

The control unit 27 is composed using a CPU (central processing unit) or the like. The control unit 27 controls processes and operations of each unit of the bio-optical measurement apparatus 2. The control unit 27 controls operations of the bio-optical measurement apparatus 2 by transferring or the like instruction information and data for each component of the bio-optical measurement apparatus 2. The control unit 27 records results of the measurement by the light-receiving unit 22 in the recording unit 25. The control unit 27 has a computation unit 271 and a drive control unit 272.

The computation unit 271 performs plural computation processes based on the results of the measurement by the light-receiving unit 22, and computes characteristic values related to the characteristics of the object to be measured. Types of the characteristic values are set in accordance with the instruction signals received by the input unit 23, for example.

The drive control unit 272, by driving each of the light source driving unit 212, the first driving unit 224, the second driving unit 225, and the third driving unit 226 based on the measurement probe 3 connected to the bio-optical measurement apparatus 2, moves the light source 211 to a position of the illumination fiber, which is in a fiber bundle composing the later-described measurement probe 3 and which emits the illumination light to the biological tissue, and also moves the first sensor unit 221, the second sensor unit 222, and the third sensor unit 223 to respective positions of a plurality of light-receiving fibers, which are in the fiber bundle and which receive returned light of the illumination light from the biological tissue at different scattering angles. Specifically, the drive control unit 272 drives each of the light source driving unit 212, the first driving unit 224, the second driving unit 225, and the third driving unit 226 based on the information read by the reading unit 26 from the recording medium attached to the measurement prove 3 connected to the bio-optical measurement apparatus 2. For example, the drive control unit 272, by driving the light source driving unit 212 based on the information read by the reading unit 26 from the recording medium attached to the measurement probe 3 connected to the bio-optical measurement apparatus 2, moves the light source 211 to cause the position of the illumination light emitted from the light source 211 to coincide with the position of the illumination fiber in the fiber bundle of the measurement probe 3. The drive control unit 272, by driving each of the first driving unit 224, the second driving unit 225, and the third driving unit 226, moves the first sensor unit 221, the second sensor unit 222, and the third sensor unit 223 to the respective positions of the light-receiving fibers of the measurement probe 3.

The measurement probe 3 will be described. The measurement probe 3 has a fiber bundle 300 formed of a plurality of optical fibers. Specifically, the measurement probe 3 is composed using a light guide (excluding an image fiber) formed by irregularly bundling the plurality of optical fibers. Being a light guide means that arrangement positions (spatial arrangements) of an optical fiber at an end face of a proximal end of the fiber bundle 300 and at an end face of a distal end of the fiber bundle 300 are different from each other. The measurement probe 3 has a proximal end 31 connected to the bio-optical measurement apparatus 2, a flexible portion 32 having flexibility, a distal end 33 that emits the illumination light supplied from the light source unit 21 and receives the returned light of the illumination light from the object S1 to be measured, an optical member 34 detachably mounted to the distal end 33, and a recording medium 35 attached to the proximal end 31.

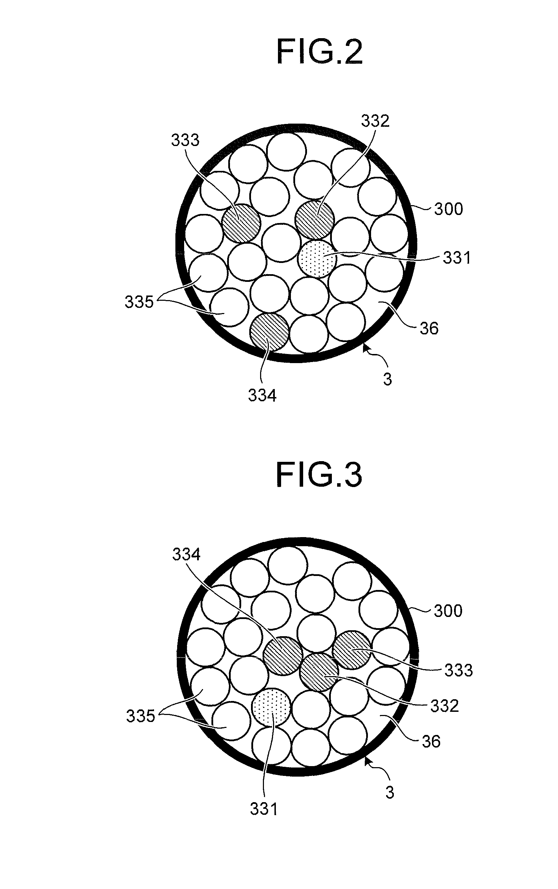

An internal configuration of the measurement probe 3 will be described in detail. FIG. 2 is a sectional view taken along a line A-A in FIG. 1. FIG. 3 is a sectional view taken along a line B-B in FIG. 1.

As illustrated in FIGS. 2 and 3, the measurement probe 3 is formed of the fiber bundle 300 formed by irregularly bundling an illumination fiber 331 that irradiates the illumination light to the object S1 to be measured, a first light-receiving fiber 332 (first light-receiving channel), a second light-receiving fiber 333 (second light-receiving channel), and a third light-receiving fiber 334 (third light-receiving channel), on which the returned light from the object S1 to be measured is incident at different angles, and other plural optical fibers 335. As illustrated in FIGS. 2 and 3, in the measurement probe 3, the respective positions of the illumination fiber 331, the first light-receiving fiber 332, the second light-receiving fiber 333, the third light-receiving fiber 334, and the other plural optical fibers 335 at the end face of the distal end 33 are different from those at the end face of the proximal end 31. At the end faces of the distal end 33 and proximal end 31, lateral faces of the illumination fiber 331, the first light-receiving fiber 332, the second light-receiving fiber 333, the third light-receiving fiber 334, and the other plural optical fibers 335 are covered by a covering member 36 for shielding light and preventing damage.

The illumination fiber 331 propagates the illumination light supplied from the light source unit 21, and irradiates the illumination light to the object S1 to be measured through the optical member 34. The number of the illumination fibers 331 may be changed as appropriate according to items to be examined and types of the object S1 to be measured, e.g., a blood flow or a locus such as a stomach, pancreas, or the like.

The first light-receiving fiber 332, the second light-receiving fiber 333, and the third light-receiving fiber 334 propagate through the optical member 34 the returned light of the illumination light from the object S1 to be measured, the returned light being incident on distal ends thereof, and outputs the propagated light to the light-receiving unit 22 from the proximal end 31. The number of the light-receiving fibers may be changed as appropriate according to items to be examined and types of the object S1 to be measured, e.g., a blood flow or a locus.

The optical member 34 is composed using glass or the like. The optical member 34 is formed to fix a distance from the illumination fiber 331 to the object S1 to be measured and to be able to irradiate light with its spatial coherent length being infallibly made constant. The optical member 34 is formed to fix a distance between the first light-receiving fiber 332 and the object S1 to be measured, a distance between the second light-receiving fiber 333 and the object S1 to be measured, and a distance between the third light-receiving fiber 334 and the object S1 to be measured, and to be able to stably receive the returned light at a predetermined scattering angle. Further, since a surface of the object S1 to be measured S1 is flattened by a bottom surface of the optical member 34, measurement of the object S1 to be measured is possible without being affected by formal irregularities of the surface of the object S1 to be measured.

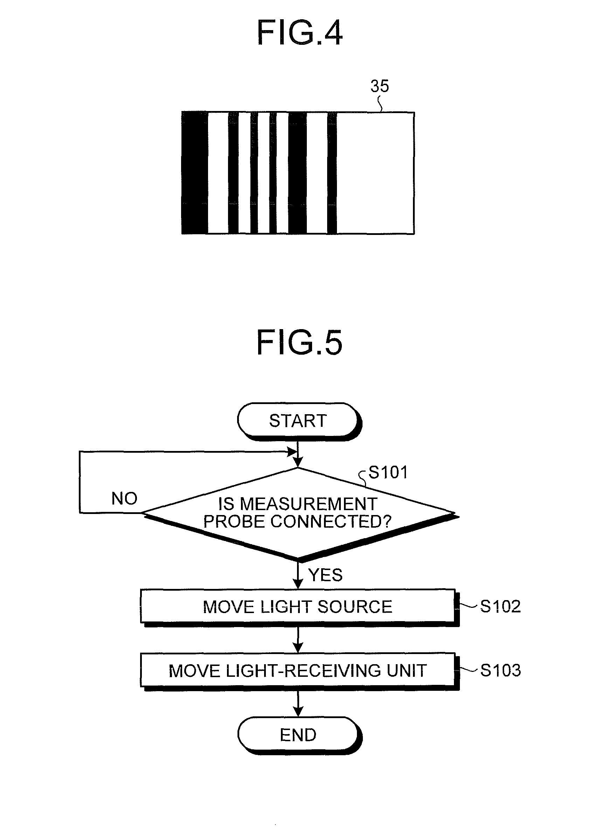

The recording medium 35 records, in association with the items to be examined for the object S1 to be measured, positional information related to the positions of an illumination fiber 331 that emits light from the light source 211 of the bio-optical measurement apparatus 2 and of a first light-receiving fiber 332, a second light-receiving fiber 333, and a third light-receiving fiber 334, which receive the returned light of the illumination light reflected and/or scattered by the object S1 to be measured at different scattering angles, the positions being in the fiber bundle 300 at both end faces of each of these fibers. In the first embodiment, the recording medium 35 is composed using a barcode as illustrated in FIG. 4. The positional information recorded on the recording medium 35 is recorded by an operator, when the measurement probe 3 is shipped from a factory. An RFID, a QR code (registered trademark), an IC chip, or the like may be used for the recording medium 35.

In the bio-optical measurement system 1 thus configured, the measurement probe 3 is inserted into a subject through a treatment tool provided to an endoscope apparatus (endoscope) of an endoscope system, the illumination fiber 331 illuminates illumination light to the object S1 to be measured, and the first light-receiving fiber 332, the second light-receiving fiber 333, and the third light-receiving fiber 334 receive the returned light from the object S1 to be measured and propagate it to the light-receiving unit 22 of the bio-optical measurement apparatus 2. Thereafter, the computation unit 271 measures characteristics of the object S1 to be measured based on results of the measurement by the light-receiving unit 22.

Next, a positioning process (calibration process) for positioning the light source unit 21 and the light-receiving unit 22 to the illumination fiber 331, the first light-receiving fiber 332, the second light-receiving fiber 333, and the third light-receiving fiber 334 of the measurement probe 3 connected to the bio-optical measurement apparatus 2 will be described. FIG. 5 is a flowchart illustrating an outline of the positioning process executed by the bio-optical measurement system 1.

As illustrated in FIG. 5, the control unit 27 determines whether the measurement probe 3 has been connected to the bio-optical measurement apparatus 2 or not (step S101). If the control unit 27 determines that the measurement probe 3 has been connected to the bio-optical measurement apparatus 2 (step 5101: Yes), step S102 is executed. On the contrary, if the control unit 27 determines that the measurement probe 3 has not been connected to the bio-optical measurement apparatus 2 (step S101: No), the bio-optical measurement system 1 repeats the determining.

Based on the positional information read by the reading unit 26 from the recording medium 35 attached to the proximal end 31 of the measurement probe 3, the drive control unit 272 drives the light source driving unit 212, thereby moving the position of the illumination light emitted from the light source 211 to coincide with the position of the illumination fiber 331 (step S102).

Thereafter, the drive control unit 272 moves the position of the light-receiving unit 22 to the position of the light-receiving fiber based on the positional information read by the reading unit 26 from the recording medium 35 attached to the proximal end 31 of the measurement probe 3 (step S103). Specifically, based on the positional information read by the reading unit 26 from the recording medium 35, the drive control unit 272 drives the first driving unit 224 thereby moving the position of the first sensor unit 221 to coincide with the position of the first light-receiving fiber 332. Further, the drive control unit 272 moves positions of the second sensor unit 222 and the third sensor unit 223 to coincide respectively with the positions of the second light-receiving fiber 333 and the third light-receiving fiber 334. After step S103, the bio-optical measurement system 1 ends this process.

According to the first embodiment of the present invention described above, the measurement probe 3 has the recording medium 35 that records therein, in association with the items to be examined for the object S1 to be measured, the positional information related to the positions of the illumination fiber 331 that emits light from the light source 211 of the bio-optical measurement apparatus 2 and of the first light-receiving fiber 332, the second light-receiving fiber 333, and the third light-receiving fiber 334, which receive the returned light of the illumination light reflected and/or scattered by the object S1 to be measured, the positions being in the fiber bundle 300 at both end faces of each of these fibers. Therefore, erroneous connection is able to be prevented with a simple structure.

Further, according to the first embodiment, even if the measurement probe 3 is made of the fiber bundle, a spatial layout (arrangement) of its optical fibers are able to be easily restricted in order to obtain predetermined properties, and therefore, cost of manufacture is able to be reduced largely.

Further, according to the first embodiment, because the measurement probe 3 is composed of the light guide, which is a generic product, the cost of manufacture is able to be reduced largely.

In the first embodiment, the recording medium 35 is attached to the proximal end 31 of the measurement probe 3, but the recording medium 35 may be attached to a box or the like that accommodates the measurement probe 3, for example. Further, the recording medium 35 may be provided inside the proximal end 31 (connector unit).

Further, in the first embodiment, the recording medium 35 is attached to the proximal end 31 of the measurement probe 3, but a tape or a recording medium having recorded thereon only identification information (device ID) of the measurement probe 3 may be attached on the recording medium 35 and the positional information corresponding to the identification information of the measurement probe 3 may be acquired through a server connected to a network.

Further, in the present first embodiment, if the item to be examined for the biological tissue recorded on the recording medium 35 and the item to be examined instructed from the input unit 23 do not coincide with each other, the control unit 27 may cause the output unit 24 to output an alarm indicating that the measurement probe 3 is unable to handle the item to be examined, which has been specified by a user.

### (Second Embodiment)

A second embodiment of the present invention will next be described. The second embodiment is different from the above-described first embodiment in its positioning of its light source unit and light-receiving unit with respect to its measurement probe. Therefore, a configuration of a bio-optical measurement system according to the second embodiment will be described, and thereafter, a process by the bio-optical measurement system according to the second embodiment will be described. Components that are the same as those in the above-described first embodiment will be described being denoted by the same reference numerals.

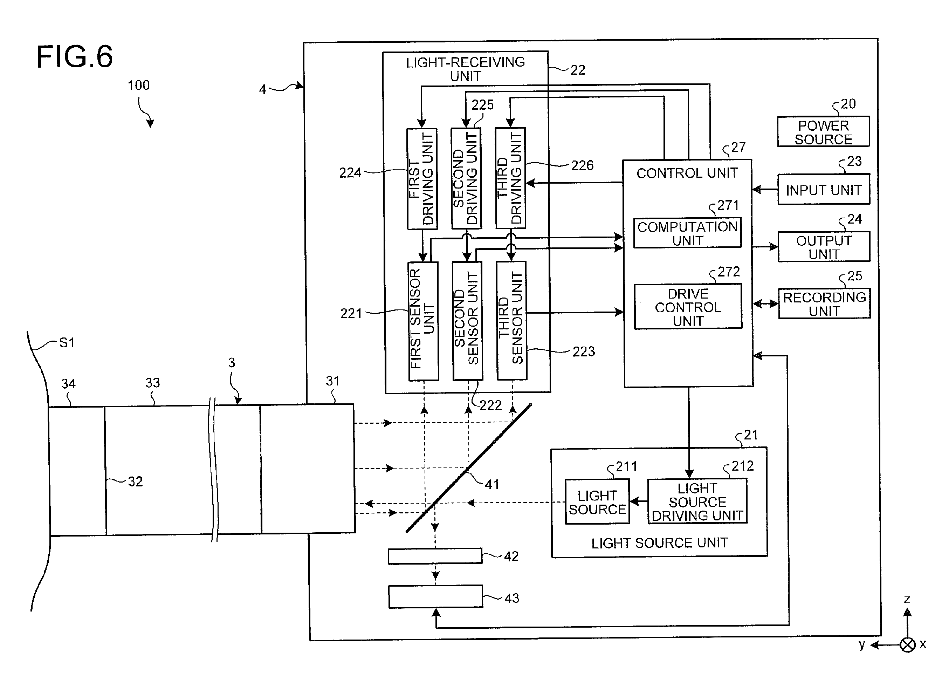

FIG. 6 is a block diagram schematically illustrating a configuration of the bio-optical measurement system according to the second embodiment. A bio-optical measurement system 100 illustrated in FIG. 6 includes a bio-optical measurement apparatus 4 that performs optical measurement with respect to an object S1 to be measured and detects characteristics of the object to be measured, and a measurement probe 3.

The bio-optical measurement apparatus 4 includes a power source 20, a light source unit 21, a light-receiving unit 22, an input unit 23, an output unit 24, a recording unit 25, a control unit 27, a half mirror 41, a fiber selecting unit 42, and an imaging unit 43.

The half mirror 41 transmits illumination light emitted from the light source unit 21 to the measurement probe 3, and reflects it toward the fiber selecting unit 42. The half mirror 41 also transmits collimated light emitted from the light source unit 21 to the measurement probe 3, and reflects it toward the fiber selecting unit 42. In this case, in a light source 211, a condensing lens has been changed to a collimator lens, or the condensing lens has been automatically moved to a position displaced from light beams of the light source 211.

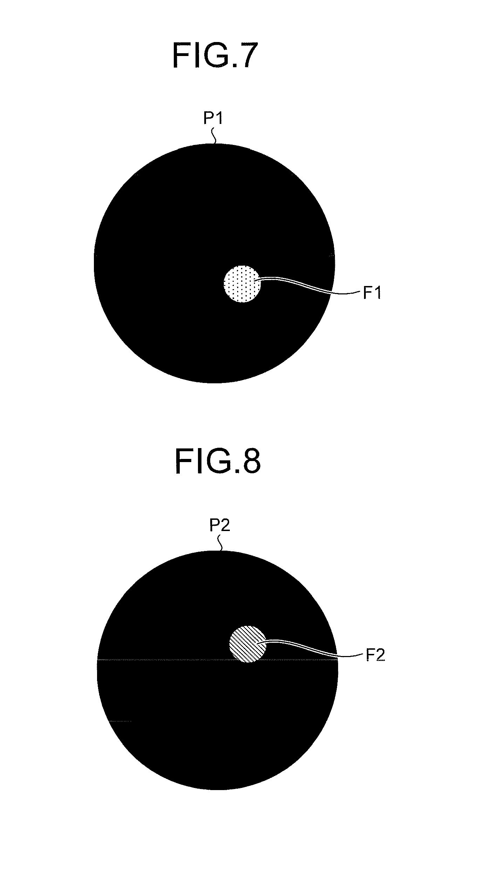

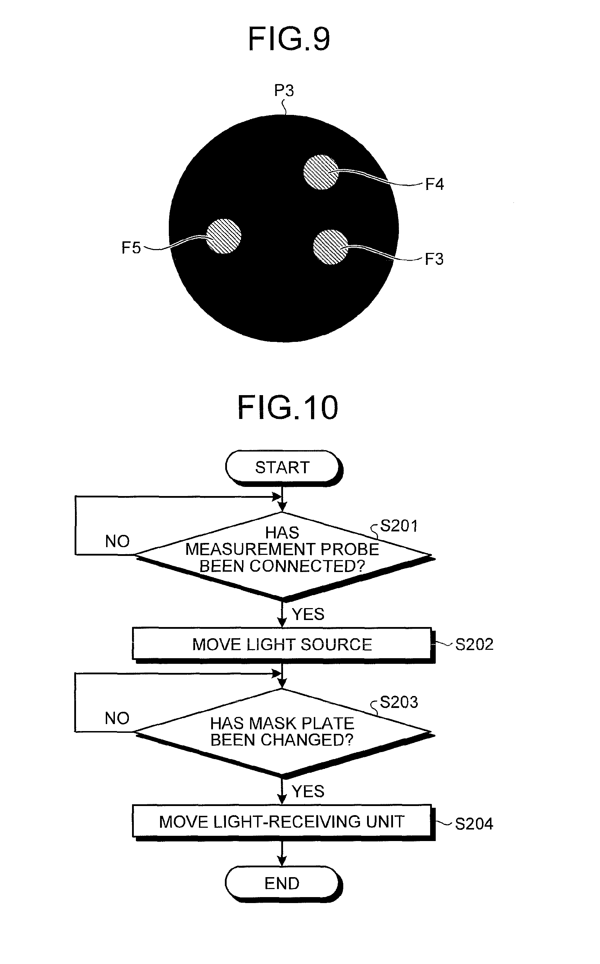

The fiber selecting unit 42 is detachable to the bio-optical measurement apparatus 4, and is composed using a mask plate that is able to select any of a plurality of optical fibers forming a fiber bundle of the measurement probe 3. Specifically, when the fiber selecting unit 42 selects the illumination fiber 331 from the fiber bundle of the measurement probe 3, a user attaches, to the bio-optical measurement apparatus 4 as illustrated in FIG. 7, a mask plate P1, which is provided with, only at a location indicating a position of an illumination fiber 331, a window (filter) F1 through which illumination light is transmittable. As a result, the illumination light transmitted through the mask plate P1 is received by the imaging unit 43. As illustrated in FIG. 8 or FIG. 9, when the fiber selecting unit 42 performs positioning of the first light-receiving fiber 332, the second light-receiving fiber 333, and the third light-receiving fiber 334, a mask plate P2, in which a window F2 that is able to transmit light is provided, or a mask plate P3 is attached. In the second embodiment, the mask plates P1 to P3 function as a recording unit. In FIG. 9, a plurality of windows F3 to F5 through which light is transmittable are provided on one mask plate P3, but they only need to be different between the illumination fiber and the light-receiving fibers.

The imaging unit 43 has an image sensor of a CCD (charge coupled device) or CMOS (complementary metal oxide semiconductor) that receives light transmitted through the fiber selecting unit 42 and converts it into an electric signal, and a signal processing unit that performs analog processing such as a noise reduction processing or gain-up processing to an analog signal output from the image sensor, performs A/D conversion thereto, and outputs to the control unit 27 positional information of the illumination fiber 331, the first light-receiving fiber 332, the second light-receiving fiber 333, and the third light-receiving fiber 334 in the fiber bundle of the measurement probe 3.

A positioning process executed by the bio-optical measurement system 100 thus configured will be described, which is for positioning the light source unit 21 and the light-receiving unit 22 respectively to the illumination fiber 331, the first light-receiving fiber 332, the second light-receiving fiber 333, and the third light-receiving fiber 334 of the measurement probe 3. FIG. 10 is a flowchart illustrating an outline of the positioning process executed by the bio-optical measurement system 100.

As illustrated in FIG. 10, the control unit 27 determines whether the measurement probe 3 has been connected to the bio-optical measurement apparatus 4 or not (step S201). When the control unit 27 determines that the measurement probe 3 has been connected to the bio-optical measurement apparatus 4 (step S201: Yes), step S202 is executed. When the control unit 27 determines that the measurement probe 3 has not been connected to the bio-optical measurement apparatus 4 (step S201: No), the bio-optical measurement system 100 repeats this determination.

Then, based on the positional information of the illumination fiber 331 in the fiber bundle of the measurement probe 3 output from the imaging unit 43, the drive control unit 272, by driving the light source driving unit 212, moves the position of the illumination light emitted from the light source 211 to coincide with the position of the illumination fiber 331 (step S202). Specifically, based on the electric signal output from the imaging unit 43, the drive control unit 272, by driving the light source driving unit 212 and scanning the fiber selecting unit 42 with the illumination light emitted from the light source 211, moves a position at which the electric signal is detected (a position at which the illumination light is transmitted) so that the position of the illumination fiber 331 coincides with the position of the illumination light from the light source unit 21.

Thereafter, the control unit 27 determines whether the mask plate has been changed or not (step S203). If the control unit 27 determines that the mask plate has been changed (step S203: Yes), step S204 is executed. If the control unit 27 determines that the mask plate has not been changed (step S203: No), the bio-optical measurement system 100 repeats this determination. In this case, the control unit 27 may cause the output unit 24 to output an alarm indicating that the mask plate is to be changed to a mask plate for a light-receiving fiber.

Next, based on the positional information on the first light-receiving fiber 332, the second light-receiving fiber 333, and the third light-receiving fiber 334 in the fiber bundle 300 of the measurement probe 3, the positional information being output from the imaging unit 43, the drive control unit 272, by driving the first driving unit 224, the second driving unit 225, and the third driving unit 226 respectively, moves positions of the first sensor unit 221, second sensor unit 222, and third sensor unit 223 in a horizontal direction and a vertical direction to coincide with the positions of the first light-receiving fiber 332, second light-receiving fiber 333, and third light-receiving fiber 334 (step S204). Thereafter, the bio-optical measurement system 100 ends this process.

According to the second embodiment of the present invention described above, because the measurement probe 3 has the mask plates P1 to P3 that associate items to be examined of an object S1 to be measured with positional information related to the positions of the illumination fiber 331 that emits light from the light source 211 of the bio-optical measurement apparatus 4 and of the first light-receiving fiber 332, the second light-receiving fiber 333, and the third light-receiving fiber 334 that receive returned light of the illumination reflected and/or scattered by the object S1 to be measured, the positions being in the fiber bundle 300 at both end faces of each of these fibers, erroneous connection is able to be prevented with a simple structure.

### Reference Signs List

1, 100 bio-optical measurement system
2, 4 bio-optical measurement apparatus
3 measurement probe
20 power source
21 light source unit
22 light-receiving unit
23 input unit
24 output unit
25 recording unit
26 reading unit
27 control unit
31 proximal end
32 flexible portion
33 distal end
34 optical member
35 recording medium
36 covering member
41 half mirror
42 fiber selecting unit
43 imaging unit
211 light source
212 light source driving unit
221 first sensor unit
222 second sensor unit
223 third sensor unit
224 first driving unit
225 second driving unit
226 third driving unit
271 computation unit
272 drive control unit
331 illumination fiber
332 first light-receiving fiber
333 second light-receiving fiber
334 third light-receiving fiber
P1, P2, P3 mask plate
S1 object to be measured

## Claims

1. A measurement probe which has a fiber bundle formed by irregularly bundling a plurality of optical fibers and which is detachably connected to a bio-optical measurement apparatus that performs optical measurement to a biological tissue that is an object to be measured, the measurement probe comprising:
a recording unit that records positional information and items to be examined of the biological tissue in association with each other, the positional information being related to each of positions of an illumination fiber that emits light from a light source of the bio-optical measurement apparatus as illumination light and of a light-receiving fiber that receives returned light of the illumination light reflected and/or scattered at the biological tissue, the positions being in the fiber bundle at both end faces of each of the illumination fiber and the light-receiving fiber.

2. The measurement probe according to claim 1, wherein the recording unit is a recording medium that is attached to the fiber bundle and from which information is readable from outside.

3. The measurement probe according to claim 1, wherein the fiber bundle is a light guide.

4. A bio-optical measurement apparatus to which a measurement probe having a fiber bundle formed by irregularly bundling a plurality of optical fibers is connectable and which irradiates illumination light to a biological tissue through the measurement probe, receives returned light of the illumination light reflected and/or scattered at the biological tissue, and performs optical measurement of the biological tissue, the bio-optical measurement apparatus comprising:
a light source that generates the illumination light to be emitted to the biological tissue through the measurement probe;
a light source driving unit that moves the light source in a predetermined direction;
a sensor unit that receives the returned light through the measurement probe;
a sensor driving unit that moves the sensor unit in a predetermined direction; and
a control unit that moves, based on the measurement probe connected to the bio-optical measurement apparatus, the light source to a position of an illumination fiber that emits the illumination light to the biological tissue, and the sensor unit to a position of a light-receiving fiber that receives the returned light, by driving the light source driving unit and the sensor driving unit respectively, the position of the illumination fiber being in the fiber bundle at both end faces of the illumination fiber, the position of the light-receiving fiber being in the fiber bundle at both end faces of the light-receiving fiber.

5. The bio-optical measurement apparatus according to claim 4, wherein
the measurement probe includes a recording unit that records positional information and items to be examined of the biological tissue in association with each other, the positional information being related to each of positions of the illumination fiber and light-receiving fiber, the positions being in the fiber bundle at both end faces of each of the illumination fiber and the light-receiving fiber,
the bio-optical measurement apparatus further comprises a reading unit that reads the positional information from the recording unit, and
the control unit drives each of the light source driving unit and the sensor driving unit based on the positional information read by the reading unit.

6. A bio-optical measurement system which includes: a measurement probe having a fiber bundle formed by irregularly bundling a plurality of optical fibers; and a bio-optical measurement apparatus to which the measurement probe is connectable and that irradiates illumination light to a biological tissue, receives returned light of the illumination light reflected and/or scattered at the biological tissue, and performs optical measurement of the biological tissue, the bio-optical measurement system comprising:
a light source that generates the illumination light to be emitted to the biological tissue through the measurement probe;
a light source driving unit that moves the light source in a predetermined direction;
a sensor unit that receives the returned light through the measurement probe;
a sensor driving unit that moves the sensor unit in a predetermined direction;
a recording unit that records positional information and items to be examined of the biological tissue in association with each other, the positional information being related to each of positions of an illumination fiber that emits light from the light source as illumination light and of a light-receiving fiber that receives the returned light, the positions being in the fiber bundle at both end faces of each of the illumination fiber and the light-receiving fiber;
a reading unit that reads the positional information from the recording unit; and
a control unit that moves, based on the positional information read by the reading unit, the light source to a position of an illumination fiber that emits the illumination light to the biological tissue, and the sensor unit to a position of a light-receiving fiber that receives the returned light, by driving the light source driving unit and the sensor driving unit respectively, the position of the illumination fiber being in the fiber bundle at both end faces of the illumination fiber, the position of the light-receiving fiber being in the fiber bundle at both end faces of the light-receiving fiber.
